# EUROPÄISCHE PATENTSCHRIFT

(11) **EP 1 583 515 B1**
(45) Veröffentlichungstag und Bekanntmachung des Hinweises auf die Patenterteilung: **23.06.2010**
(21) Anmeldenummer: 04702697.6
(22) Anmeldetag: 16.01.2004
(51) Int. Cl.: A61Q 19/10, A61K 8/02, A61K 8/26, A61K 8/36, A61Q 3/00, A61Q 19/00

(54) **KOSMETISCHE SELBSTERWÄRMENDE PRODUKTE**
SELF-HEATING COSMETIC PRODUCTS
PRODUITS COSMETIQUES AUTOCHAUFFANTS

(30) Priorität: 16.01.2003 DE 10302096
(43) Veröffentlichungstag der Anmeldung: 12.10.2005
(73) Patentinhaber: Coty B.V., 2031 CC Haarlem (NL)
(72) Erfinder: HWANG, Donna, Hui-Ing, Leonia, NJ 07605 (US); CERNASOV, Dominica, Ringwood, NJ 07456 (US); MACCHIO, Ralph, Sparta, NJ 07971 (US)
(74) Vertreter: Walter, Wolf-Jürgen
(86) Internationale Anmeldenummer: PCT/EP2004/000317
(87) Internationale Veröffentlichungsnummer: WO 2004/062636

(56) Entgegenhaltungen:
- EP-A- 1 172 088
- WO-A-86/05389
- PATENT ABSTRACTS OF JAPAN Bd. 016, Nr. 315 (C-0961), 10. Juli 1992 (1992-07-10) & JP 04 089424 A (TOUYOU EAZOORU KOUGIYOU KK), 23. März 1992 (1992-03-23)

## Beschreibung

Die Erfindung betrifft kosmetische Produkte mit Selbsterwärmungseffekt gemäß Anspruch 1.

Aus der US-A-6309655 ist eine kosmetische Zusammensetzung bekannt, die eine sich selbst erwärmende Komponente enthält und aus einem den Zerfall selbstanzeigenden Granulat besteht, wobei das Granulat eine wasserunlösliches Polymeres, einen Farbstoff und einen wasserfreien, mit Wasser mischbaren Träger enthält. Das wasserlösliche Polymere ist z.B. ein Polyethylen, Polystyren, Polyvinylchlorid, Polyacrylharz usw., und der mit wasser mischbare Träger ein mehrwertiger Alkohol, ein Zuckeralkohol und Ethylenaddukte verschiedener Zucker und/oder ein Zeolith.

Die WO 00/38621 beschreibt ein selbsterwärmendes Haarkonditionierungsmittel, bestehend aus einem mikroporösen Material mit Porengrößen größer als Wassermoleküle, wie z.B. ein Alkalisilicat, Bentonit, Diatomeenerde, ein Trägermolekül mit einem kritischen Durchmesser größer als das mikroporöse Material, wie z.B. hydrophile Glycole, Polyethylenglycole, Glycerin usw. und ein Konditionierungsmolekül mit einem Durchmesser größer als das mikroporöse Material, wie z.B. quaternäre Ammoniumverbindungen, kationische Polymere und höhere Kohlenwasserstoffe.

Aus der WO 86/05389 ist ein wasserfreier Aerosolschaum bekannt, der in einem flüssigen Öl dispergierte aluminiumarme Zeolithteilchen enthalten kann. Die EP 1172088 beschreibt exotherme kosmetische Zusammensetzungen mit Zeolithen, die mehrwertige Alkohole enthalten. Die Pyrogenzusammensetzung aus der JP 04089424 enthält neben Zeolithen ebenfalls mehrwertige Alkohole.

Der Erfindung liegt die Aufgabe zugrunde, ein kosmetisches Selbsterwärmungsprodukt mit verbesserter Wasserabsorption und damit verstärkter Erwärmungswirkung bereitzustellen.

Eine weitere Aufgabe besteht darin, ein Produkt mit besonders weichem, seidenartigen Nachgefühl zu entwickeln. Eine weitere Aufgabe besteht darin, ein Reinigungsprodukt mit Selbsterwärmungswirkung zu entwickeln.

Erfindungsgemäß bereitgestellt wird ein kosmetisches selbsterwärmendes Produkt gemäß Anspruch 1.

Gegenüber bekannten kosmetischen Produkten wird durch das Vorhandensein von Zeolithen und der bei Kontakt mit Wasser auftretenden Erwärmung eine Öffnung der Hautporen bewirkt, wodurch das als Konditionierungs/Reinigungsmittel eingesetzte Öl wirksamer arbeiten kann. Gegenüber bekannten kosmetischen Selbsterwärmungsprodukten, die generell mehrwertige Alkohole und Zeolithe enthalten, wird durch das Fehlen mehrwertiger Alkohole das Potential zur Wasserabsorption deutlich herabgesetzt. Dadurch kann der im Produkt vorhandene Zeolith voll in dem Moment wärmewirksam werden, wo dies beabsichtigt ist und nicht bereits vorher. Bei einem hohen Wasserabsorptionspotential durch andere Bestandteile kann bereits vor der eigentlichen Anwendung ein Zutritt von wenigen Zehntel Prozent Wasser dazu führen, daß eine Erwärmung des Zeoliths teilweise dessen volles Erwärmungspotential herabsetzt.

Eine besondere Tiefen-Wärmewirkung wird durch das erfindungsgemäße Produkt erreicht und ein spezielles seidenartiges Gefühl nach dem Auftragen des Produktes auf die Haut.

Je mehr Zeolith durch die exotherme Reaktion mit Wasser wärmewirksam wird, desto besser ist der Porenöffnungseffekt und desto wirksamer tritt die Konditionierung- und/oder Reinigungswirkung ein. Durch die Kombination eines hohen Ölanteils in dem erfindungsgemäßen Mittel mit dem Zeolith und dem Fehlen mehrwertiger Alkohole wird auch ein gewisser wasserabweisender Effekt erzielt, der dazu beiträgt, daß das Produkt äußerst lagerstabil bei Erhaltung aller angestrebten Eigenschaften ist.

Das erfindungsgemäße Produkt kann zusätzlich in Mengen im Bereich von 0,1 bis 20 Gew-%, bezogen auf das Gesamtgewicht des Produktes, vorteilhaft ein Ölabsorbens enthalten, um die Klebrigkeit oder ein Fettgefühl herabzusetzen. Es können daher Zinkstearat, Kaolin, Kaolin gemäß WO96/17588 Aluminium-Stärke-octenylsuccinat und ähnliche Produkte enthalten sein.

Kaolin gemäß WO96/17588 ist ein Kaolin, der mit sphärischen TiO₂- oder SiO₂-Teilchen mit einer Teilchengröße <5 µm im modifiziert ist, wobei die sphärischen Teilchen einen Anteil an der Kaolinmischung von 0,5 bis 10 Gew-% haben.

Es ist weiterhin vorteilhaft, ein Gleitmodifikator in Mengen im Bereich von 0,1 bis 20 Gew-% einzubringen, bezogen auf das Gesamtgewicht des Produktes, wie z.B. Talkum, Silica, Trimethylsiloxysilicat usw. Dadurch erhält das Produkt eine sehr gute taktile Ästhetik und ein sehr angenehmes Gefühl beim Auftragen auf die Haut.

Bei Kontakt mit Wasser tritt der beste Effekt mit etwa 50 Gew-% Wasser im Verhältnis zum Gewicht des verwendeten erfindungsgemäßen Produkts ein. Der Erwärmungseffekt ist jedoch schon bei 0,5 Gew-% Wasser fühlbar und ist noch fühlbar mit etwa 300 Gew-% Wasserzugabe im Verhältnis zum Gewicht des erfindungsgemäßen Produktes. Die Erwärmung durch den Zeolith führt dabei zu Temperaturhöhungen von 4 bis 8 K gegenüber der Oberflächentemperatur der zu behandelnden Haut.

Der eingesetzte Zeolith liegt in vollständig trockener Form vor. Es ist vorteilhaft, einen aluminiumreichen, hydrophilen synthetischen Zeolith einzusetzen, wie Zeolith A, NaX oder NaY, oder einen natürlichen Zeolith mit entsprechenden Eigenschaften. Ein bevorzugter alumiumreicher Zeolith hat ein Verhältnis Si/Al im Bereich von 2 bis 5:1. Die mittlere Porengröße des Zeoliths liegt allgemeinen im Bereich von 3 bis 5 Angström, um das Eintreten von Wassermolekülen in die Poren zu ermöglichen.

Ein bevorzugter Bereich für den Zeolithgehalt im Produkt sind 15 bis 25 Gew-% oder 15-28 Gew-%.

Die disperse Ölphase kann in dem vorliegenden kosmetischen Produkt durch unpolare, halbpolare und polare Öle gebildet werden, ausgewählt aus Triglyceriden, Siliconölen, oder Gemischen davon. Ein bevorzugtes disperses Öl ist ein solches, das die Oberflächenspannung des Zeoliths herabsetzt, wie Caprylic/Capric Triglyceride. Alle verwendeten Öle sind wasserfreie Öle.

Es ist weiterhin bevorzugt, einen hohen Ölgehalt von 20 bis 70 Gew-% in der Formulierung zu haben, insbesondere einen Ölgehalt von 25 bis 50 Gew-%. Das wasserfreie disperse Öl wirkt auch Dispergiermittel, um die Leistungsfähigkeit und die ästhetische Effizienz des Zeoliths in der Formulierung zu erreichen, ohne daß Scherkraft für eine gute Dispersion eingesetzt werden müßte. Da Zeolith in Puderform in den meisten Lösungsmitteln nicht löslich ist, ist das Erreichen einer guten Suspension meist ein Problem. Normalerweise ist ein hoher Energie-Eintrag in Form von Scherkraft erforderlich, um eine solche gute Suspendierung zu erreichen. Dies wird durch das spezielle disperse Öl vermieden.

Das nichtionische oberflächenaktive Mittel ist ein Ester, Ether, Alkanolamid oder Aminoxid, bevorzugt ein Sorbitanester, Trialkylphosphat, ethoxylierter Alkohol, ethoxyliertes Rizi-nusöl, ethoxyliertes Polysiloxan, Alkylglycosid, ein POE/PPG-Ester, Palmitamid-DEA, Dihydroxyethylstearaminoxid, methoxyliertes Glycerid, Lecithin usw.

Das nichtionische oberflächenaktive Mittel wie z.B. Tween 60 oder Lecithin ermöglicht ein besseres Abspülen der Reinigungszusammensetzung von der Haut.

Die bevorzugte Menge für das oberflächenaktive Mittel liegt im Bereich von 3 bis 15 Gew-%.

Die Zusammensetzung enthält weiterhin 0,1 bis 40 Gew-%, vorzugsweise 10 bis 25 Gew-% eines Erweichungsmittels. Solche Erweichungsmittel sind z.B. Ester von -Fettsäuren mit 12-18 Kohlenstoffatomen im Alkylteil der Säure. Der Alkoholrest ist vorteilhaft ein geradkettiger, verzweigter oder cyclischer Kohlenwasserstoff mit 10-40 Kohlenstoffatomen, ein Glycerylester oder ein niedermolekulares α-Olefin. Beispiel dafür sind Glycerylmonoricinoleat, Glycerylmonostearat, Isopropylisostearat, Isobutylpalmitat, Isopropyllaurat, Isopropylmyristat, Decyloleat, Cetylpalmitat; oder auch Siliconöle wie Dimethylpolysiloxan, oder Lanolin und Kakaobutter.

Die Zusammensetzung enthält weiterhin 0,1 bis 20 Gew-%, vorzugsweise 0,2 bis 10 Gew-% eines Verdickungsmittels. Das verdickungsmittel kann die Viskosität des Öles modifizieren und erhöhen und die Struktur der Phase aufbauen. Als Verdickungsmittel werden Polyethylen, 12-Hydroxystearinsäure, Silicon-Copolymere, Ton, Rizinusölderivate und Wachse eingesetzt. Beispiele dafür sind Polyethylen mit Molekulargewichten im Bereich von 300 bis 600 und Wachse wie Carnaubawachs, Candellilawachs, Bienenwachs, Wollwachs, Hartparaffin, Ceresin, Ozokerit, Silicone, mikrokristalliner Wachs oder Polyethylenglycolesterwachse. Bevorzugte Verdickungsmittel sind Wachs und Rizinusölderivate.

Das erfindungsgemäße Produkt enthält weiterhin kosmetische Hilfs- und Trägerstoffe, wie sie üblicherweise in solchen Zubereitungen verwendet werden, z.B. Konservierungsmittel, Farbstoffe, Duftstoffe, Gelbildner, Polymere, Copolymere, Emulgatoren, Stabilisatoren.

Zu den ebenfalls einsetzbaren kosmetischen Wirkstoffen gehören z. B. anorganische und organische Lichtschutzmittel, Radikalfänger, Feuchthaltemittel, Vitamine, Enzyme, pflanzliche Wirkstoffe, Antioxidationsmittel, entzündungswidrige natürliche Wirkstoffe.

Die Verwendung des erfindungsgemäßen kosmetischen Produktes kann erfolgen in einer Reinigungsmilch, in Gesichtsmasken, Haarmasken, Lotionen und anderen Reinigungszusammensetzungen. Die Herstellung derartiger Produkte erfolgt auf eine Weise, wie sie dem Fachmann auf diesem Gebiet bekannt ist.

Die Erfindung soll nachstehend durch Beispiele näher erläutert werden. Alle Angaben erfolgen in Gewichtsprozent, sofern nichts anderes angegeben ist.

### Beispiel 1 Erwärmungsbalsam

| | Ingredienzen | Gew-% |
|---|---|---|
| A | caprylic/Capric Triglyceride | 35-40 |
| | C18-36 Acid Glycolester | 2-5 |
| | synthetischer wachs | 2-5 |
| B | Isopropyl Palmitate | 20-25 |
| | Zeolith | 18-22 |
| | Pigment | qs. |
| C | Schutzmittel | qs. |
| | PPG-15 Stearyl Ether | 8-12 |
| D | Tocopheryl Acetate | qs. |
| | Parfumol | qs. |
| | **Total** | 100,0 |

| | | |
|---|---|---|
| ¹ Kaliumform von Zeolith A (Molsiv^{®} GMP3A von UOP LLC, Des Plaines, IL. NJ, USA)) | | |

Alle Bestandteile der Phase A werden in einen sauberen, trockenen Behälter aus rostfreiem Stahl gegeben, der mit einem Rührer ausgestattet ist. Unter langsamem Rühren wird das Gemisch auf ca. 80°C erhitzt und die Temperatur gehalten bis das Gemisch gleichförmig ist und keine ungelösten Rohmaterialien mehr enthält. Separat werden die Ingredienzen der Phase B gleichmäßig vermischt und dann Phase B zu Phase A bei moderatem Mischen gegeben. Es wird 15 Minuten oder erforderlichenfalls länger gemischt, bis ein gleimäßiges Gemisch erhalten wird. Unter forlaufendem Mischen werden die Ingredienzen der Phase C zu den Phasen A+B gegeben und die Temperatur bei 70-75°C bis zum gleichmäßigen Vermischen gehalten. Danach wird auf etwa 50°C abgekühlt. Bei Erreichen der Temperatur von 50°C wird das Parfümöl zugegeben. Danach wird wieder bis zum gleichmäßigen Gemisch und bis zur Homogenität gerührt.

### Beispiel 2 Wärmebildende Creme

| | **Ingredienzen** | **Gew- %** |
|---|---|---|
| A | Caprylic/Capric Triglyceride | 30-35 |
| | Trihydroxystearin | 0.5-1 |
| B | mikrokristalliner Wachs | 2-5 |
| | C18-36 Acid Glycol Ester | 0.5-1 |
| | Tribehenin | 2-4 |
| | BHT (Dibutylhydroxytoluen) | qs. |
| | Schutzmittel | qs. |
| C | Isopropyl Palmitate | 15-20 |
| | Maisstarke | 10-15 |
| | Zeolith | 20-25 |
| | Pigment | qs. |
| D | Tocopheryl acetate | qs. |
| | Parfumol | qs. |
| | **Total** | 100,0 |

Alle Bestandteile der Phase A werden in einen sauberen, trocknen Behälter aus rostfreiem Stahl gegeben, der mit einem Rührer ausgestattet ist. Unter langsamem Rühren wird das Gemisch auf ca. 80°C erhitzt und die Temperatur gehalten bis das Gemisch gleichförmig ist und keine ungelösten Rohmaterialien mehr enthält. Separat werden die Ingredienzen der Phase B gleichmäßig vermischt und dann Phase B zu Phase A bei moderatem Mischen gegeben. Es wird 15 Minuten oder erforderlichenfalls länger gemischt, bis ein gleichmäßiges Gemisch erhalten wird. Unter forlaufendem Mischen werden die Ingredienzen der Phase C zu den Phasen A+B gegeben und die Temperatur bei 70-75°C bis zum gleichmäßigen Vermischen gehalten. Danach wird auf etwa 50°C abgekühlt. Bei Erreichen der Temperatur von 50°C wird das Parfümöl zugegeben. Danach wird wieder bis zum gleichmäßigen Gemisch und bis zur Homogenität gerührt.

### Beispiel 3 Erwärmungs-Fußreiniger

| | **Ingredienzen** | **Gew-%** |
|---|---|---|
| A | Caprylic/Capric Triglyceride | 28-33 |
| | Trihydroxystearin | 3-6 |
| B | Polyethylene | 1-3 |
| | synthetischer wachs | 1-5 |
| | C18-36 Acid Glycol Ester | 1-5 |
| | BHT | qs. |
| C | Caprylic/Capric Triglyceride | 8-12 |
| | Isopropyl Palmitate | 8-15 |
| | Quarz und Bimsstein | 6-10 |
| | Zeolith | 15-22 |
| D | Polysorbate 60 | 6-10 |
| E | Schutzmittel | qs. |
| | Parfumol | qs. |
| | **Total** | 100,0 |

Alle Bestandteile der Phase A werden in einen sauberen, trockenen Behälter aus rostfreiem Stahl gegeben, der mit einem Rührer ausgestattet ist. Unter langsamem Rühren wird das Gemisch auf ca. 80°C erhitzt und die Temperatur gehalten bis das Gemisch gleichförmig ist und keine ungelösten Rohmaterialien mehr enthält. Unter fortwährendem Mischen werden die Ingredienzen der Phase B zur Phase A gegeben und die Temperatur bei 80 °C gehalten. Separat wird die Phase C hergestellt, und unter fortlaufendem Mischen wird die Phase C zu den Phasen A+B gegeben und darin weiter 15 Minuten gemischt. Unter fortwährendem Mischen werden die Ingredienzen der Phase D zu den Phasen A+B+C gegeben und die Temperatur bei 80 EC gehalten. Danach wird auf etwa 50°C abgekühlt. Bei Erreichen der Temperatur von 50°C werden Parfümöl und Schutzmittel zugegeben.- Danach wird wieder bis zum gleichmäßigen Gemisch und bis zur Homogenität gerührt.

### Beispiel 4 Erwärmende Reinigungslotion

| | **Ingredienzen** | **Gew-%** |
|---|---|---|
| A | Caprylic/Capric Triglyceride | 35-40 |
| | Trihydroxystearin | 3-6 |
| B | Polyethylene | 1-3 |
| | BHt | qs. |
| C | Caprylic/Capric Triglyceride | 9-16 |
| | Triethyl Hexanoin | 4-6 |
| | Polyglyceryl-3-laurate | 4-6 |
| | Zeolith | 20-25 |
| | Kaolin | 1-3 |
| D | Polysorbate 60 | 4-8 |
| | Lecithin | 1-3 |
| E | Schutzmittel | qs. |
| | Parfumol | qs. |
| | **Total** | 100,0 |

| | | |
|---|---|---|
| ¹ Natriumform von Zeolith A (Molsiv^{®} GMP 4A von UOP, Mt. Laurel, NJ, USA) | | |

Alle Bestandteile der Phase A werden in einen sauberen, trockenen Behälter aus rostfreiem Stahl gegeben, der mit einem Rührer ausgestattet ist. Unter langsamem Rühren wird das Gemisch auf ca. 80°C erhitzt und die Temperatur gehalten bis das Gemisch gleichförmig ist und keine ungelösten Rohmaterialien mehr enthält. Unter fortwährendem Mischen werden die Ingredienzen der Phase B zur Phase A gegeben und die Temperatur bei 80 °C gehalten. Separat wird die Phase C hergestellt, und unter fortlaufendem Mischen wird die Phase C zu den Phasen A+B gegeben und dann weiter 15 Minuten gemischt. Unter fortwährendem Mischen werden die Ingredienzen der Phase D zu den Phasen A+B+C gegeben und die Temperatur bei 80 °C gehalten. Danach wird auf etwa 50°C abgekühlt. Bei Erreichen der Temperatur von 50°C werden Parfümöl und Schutzmittel zugegeben. Danach wird wieder bis zum gleichmäßigen Gemisch und bis zur Homogenität gerührt.

## Patentansprüche

1. Kosmetisches selbsterwärmendes Produkt zur Hautreinigung, **dadurch gekennzeichnet, daß** das Produkt als Reinigungsmilch, Handcreme, Gesichtsmaske, Lotion oder Fuß-Reinigungsprodukt vorliegt und enthält
15 bis 40 Gew-% eines hydrophilen, vollständig trockenen, aluminiumreichen Zeoliths mit einer mittleren Porengröße von 0,3 bis 0,5 nm,
20 bis 70 Gew-% einer dispersen Ölphase, ausgewählt aus der Gruppe, bestehend aus Triglyceriden, Siliconölen und Gemischen davon,
0,1 bis 50 Gew-% eines nichtionischen oberflächenaktiven Mittels,
0,1 bis 40 Gew-% eines Erweichungsmittels,
0,1 bis 20 Gew-% eines Verdickungsmittels, ausgewählt aus der Gruppe, bestehend aus Polyethylen, 12-Hydroxystearinsäure, Ton, Rizinusölderivaten und Wachsen, und
der Rest zu 100 Gew% kosmetische Trägerstoffe, Hilfsstoffe, Wirkstoffe und Gemische davon,
mit der Maßgabe, daß keine mehrwertigen Alkohole und polaren Lösungsmittel enthalten sind und dass außer gegebenenfalls Kristallwasser von Ingredienzien kein Wasser enthalten ist.

2. Produkt nach Anspruch 1, **dadurch gekennzeichnet, daß** es ein Ölabsorbens enthält, ausgewählt aus der Gruppe, bestehend aus Zinkstearat, Kaolin und Gemischen davon.

3. Produkt nach Anspruch 1, **dadurch gekennzeichnet, daß** es einen Zeolith mit einem Verhältnis Si/Al im Bereich von 2-5:1 enthält.

4. Produkt nach Anspruch 1, **dadurch gekennzeichnet, daß** der Zeolithgehalt im Bereich von 15 bis 25 Gew-% liegt.

5. Produkt nach Anspruch 1, **dadurch gekennzeichnet, daß** das disperse Öl wasserfreies Caprylic/Capric Triglyceride ist.

6. Produkt nach Anspruch 1, **dadurch gekennzeichnet, dass** das Verdickungsmittel 0,2 bis 10 Gew-% Wachs oder Rizinusölderivat oder ein Gemisch davon ist.

7. Verwendung eines Mittels, bestehend aus 15 bis 40 Gew-% eines hydrophilen, vollständig trockenen, aluminiumreichen Zeoliths mit einer mittleren Porengröße von 0,3 bis 0,5 nm, 20 bis 70 Gew-% einer dispersen Ölphase, ausgewählt aus der Gruppe, bestehend aus Triglyceriden, Siliconölen und Gemischen davon, 0,1 bis 50 Gew-% eines nichtionischen oberflächenaktiven Mittels, 0,1 bis 40 Gew-% eines Erweichungsmittels, 0,1 bis 20 Gew-% eines Verdickungsmittels, ausgewählt aus der Gruppe, bestehend aus Polyethylen, 12-Hydroxystearinsäure, Ton, Rizinusölderivaten und Wachsen, und der Rest zu 100 Gew% kosmetische Trägerstoffe, Hilfsstoffe, Wirkstoffe und Gemische davon, mit der Maßgabe, daß keine mehrwertigen Alkohole und polaren Lösungsmittel enthalten sind,
zusammen mit 10 bis 100 Gew-% Wasser, bezogen auf das Gewicht des Produkts, zur Tiefenreinigung der Haut mit einer Temperaturerhöhung gegenüber der Oberflächentemperatur der Haut von 4 bis 8 K in Form einer Reinigungsmilch, Handcreme, Gesichtsmaske, Lotion oder eines Fuß-Reinigungsproduktes.

8. Verwendung nach Anspruch 7 in Verbindung mit 40 bis 50 Gew-% Wasser.

## Claims

1. A cosmetic self-warming product for skin cleansing, wherein the product is a cleansing milk, hand cream, face mask, lotion or foot scrub, which product contains
15 to 40 % by weight of a hydrophilic, completely dry aluminum-rich zeolite with a medium pore size in the range of 0.3 to 0.5 nm,
20 to 70 % by weight of a disperse oil phase, selected from the group consisting of triglycerides, silicone oils and mixtures thereof,
0.1 to 50 % by weight of a non-ionic surface-active agent,
0.1 to 40 % by weight of an emollient,
0.1 to 20 % by weight of a thickener, selected from the group consisting of polyethylene, 12-hydroxy stearic acid, clay, castor oil derivatives and waxes, and
ad 100 % by weight cosmetic carrier substances, auxiliary substances, active substances and mixtures thereof,
with the proviso that no polyvalent alcohols and polar solvents are contained and that no water is contained apart from possibly crystal water from ingredients.

2. A product according to Claim 1 wherein the product contains an oil absorbent selected from the group consisting of zinc stearate, kaolin and mixtures thereof.

3. A product according to Claim 1 wherein the product contains a zeolite with a ratio Si/Al in the range of 2-5:1.

4. A product according to Claim 1 wherein the zeolite content is in the range of 15 to 25 % by weight.

5. A product according to Claim 1 wherein the dispersed oil is anhydrous Caprylic/Capric Triglyceride.

6. A product according to Claim 1 wherein the thickener is 0.2 to 10 % by weight wax or castor oil derivative or a mixture thereof.

7. The use of a product consisting of
15 to 40 % by weight of a hydrophilic, completely dry aluminum-rich zeolite with a medium pore size in the range of 0.3 to 0.5 nm,
20 to 70 % by weight of a disperse oil phase selected from the group consisting of triglycerides, silicone oils and mixtures thereof,
0.1 to 50 % by weight of a non-ionic surface-active agent,
0.1 to 40 % by weight of an emollient,
0.1 to 20 % by weight of a thickener selected from the group consisting of polyethylene, 12-hydroxy stearic acid, clay, castor oil derivatives and waxes, and
ad 100 % by weight cosmetic carrier substances, auxiliary substances, active substances and mixtures thereof,
with the proviso that no polyvalent alcohols and polar solvents are contained,
in combination with 10 to 100 % by weight of water relative to the weight of the product for thorough cleansing of the skin during which the temperature increases by 4 to 8 K compared to the surface temperature of the skin in the form of a cleansing milk, a hand cream, face mask, lotion or foot scrub.

8. Use according to Claim 7 in combination with 40 to 50 % by weight water.

## Revendications

1. Produit cosmétique auto-chauffant servant à nettoyer la peau, **caractérisé en ce que** le produit se présente sous la forme d'un lait nettoyant, d'une crème pour les mains, d'un masque pour le visage, d'une lotion ou d'un produit nettoyant pour les pieds, et **en ce qu'**il contient
15 à 40 % en poids d'une zéolithe hydrophile, complètement sèche et riche en aluminium, présentant une grosseur de pore moyenne allant de 0,3 nm 0,5 nm,
20 à 70 % en poids d'une phase dispersée huileuse, choisie parmi le groupe se constituant de triglycérides, d'huiles de silicone et d'un mélange de ces derniers,
0,1 à 50 % en poids d'un agent non ionique tensio-actif,
0,1 à 40 % en poids d'un agent de ramollissement,
0,1 à 20 % en poids d'un épaississant, choisi parmi le groupe se constituant du groupe composé de polyéthylène, d'acide hydroxy-12 stéarique, d'argile, de dérivés d'huile de ricin et de cires, et
**en ce que** le reste contient jusqu'à 100 % en poids d'excipients cosmétiques, d'adjuvants, de principes actifs et de mélanges de ces derniers,
étant précisé qu'aucun alcool polyvalent et qu'aucun solvant polaire ne sont présents et qu'à l'exception de l'eau de constitution éventuellement présente dans les ingrédients aucune eau n'est présente.

2. Produit selon la revendication 1, **caractérisé en ce qu'**il contient un absorbant d'huile, choisi parmi le groupe se constituant de stéarate de zinc, de kaolin et de mélanges de ceux-ci.

3. Produit selon la revendication 1, **caractérisé en ce qu'**il contient une zéolithe avec un rapport Si/Al se trouvant dans la plage allant de 2 à 5:1.

4. Produit selon la revendication 1, **caractérisé en ce que** la teneur en zéolithe se situe dans la plage allant de 15 à 25 % en poids.

5. Produit selon la revendication 1, **caractérisé en ce que** l'huile dispersée est Caprylic/Capric Triglyceride exempt d'eau.

6. Produit selon la revendication 1, **caractérisé en ce que** l'agent épaississant contient 0,2 à 10 % en poids de cire ou d'un dérivé d'huile de ricin ou d'un mélange de ceux-ci.

7. Utilisation d'un agent se composant de 15 à 40 % en poids d'une zéolithe hydrophile, complètement sèche et riche en aluminium présentant une grosseur de pore moyenne allant de 0,3 à 0,5 nm, de 20 à 70 % en poids d'une phase dispersée huileuse, choisie parmi le groupe se constituant de triglycérides, d'huiles de silicone et de mélanges de ces derniers, de 0,1 à 50 % en poids d'un agent non ionique tensio-actif, de 0,1 à 40 % en poids d'un agent de ramollissement, de 0,1 à 20 % en poids d'un agent épaississant choisi parmi le groupe se constituant de polyéthylène, d'acide hydroxy-12 stéarique, d'argile, de dérivés d'huile de ricin et de cires, sachant que le reste contient jusqu'à 100 % en poids d'excipients cosmétiques, d'adjuvants, de principes actifs et de mélanges de ces derniers, étant précisé qu'aucun alcool polyvalent ou solvant polaire ne sont présents,
le tout étant combiné avec 10 à 100 % en poids d'eau, par rapport au poids du produit, servant au nettoyage en profondeur
le tout étant combiné avec 10 à 100 % en poids d'eau, par rapport au poids du produit, servant au nettoyage en profondeur de la peau avec une élévation de température par rapport à la température de la peau en surface de 4 à 8 K sous la forme d'un lait nettoyant, d'une crème pour les mains, d'un masque pour le visage, d'une lotion ou d'un produit nettoyant pour les pieds.

8. Utilisation selon la revendication 7 en relation avec 40 à 50 % en poids d'eau.
